# EUROPÄISCHE PATENTSCHRIFT

(11) **EP 0 971 669 B1**
(45) Veröffentlichungstag und Bekanntmachung des Hinweises auf die Patenterteilung: **25.09.2002**
(21) Anmeldenummer: 98919220.8
(22) Anmeldetag: 03.04.1998
(51) Int. Cl.: A61F 13/20, A61K 9/02

(54) **VAGINALTAMPON**
VAGINAL TAMPON
TAMPON VAGINAL

(30) Priorität: 04.04.1997 DE 19713908
(43) Veröffentlichungstag der Anmeldung: 19.01.2000
(73) Patentinhaber: Symbio Herborn Group GmbH & Co., 35745 Herborn-Hörbach (DE)
(72) Erfinder: RUSCH, Volker, D-35745 Herborn (DE); ZIMMERMANN, Kurt, D-35745 Herbornseelbach (DE); BRUNSMANN, Holger, D-35745 Herborn (DE); SOLFRONK, Joachim, Bruno, D-61350 Bad Homburg (DE)
(74) Vertreter: Pätzold, Herbert, Dr.-Ing.
(86) Internationale Anmeldenummer: EP9801969
(87) Internationale Veröffentlichungsnummer: WO98044884

(56) Entgegenhaltungen:
- EP-A- 0 415 087
- WO-A-92/13577
- DE-A- 1 807 734
- DE-A- 2 041 242
- DE-A- 3 545 926

## Beschreibung

Die Erfindung bezieht sich auf einen Vaginaltampon und Verfahren zu seiner Herstellung.

Der Vaginalbereich gesunder Frauen weist ein milchsaures Milieu auf. Die Ursache von Erkrankungen im Vaginal- und Vulvenbereich ist vielfach eine gestörte Vaginalflora. Ursache oder Folge hiervon ist auch ein erhöhter pH-Wert. Es sind Zäpfchen zur Behandlung der Vaginalmuscosa bekannt. Nachteilig ist dabei, daß nur ein relativ kleiner Teil der Schleimhäute mit dem Zäpfchen bzw. dem von ihm freigesetzten Stoffen in Kontakt kommt, so daß der prophylaktische oder therapeutische Erfolg unbefriedigend ist.

Es besteht daher ein Bedürfnis, einfach zu handhabende Mittel zur Anwendung im Intimbereich von Frauen zur Verfügung zu haben, die eine leichte Pflege und Stützung einer gesunden Vaginalflora oder auch eine Regenerierung einer geschwächten oder verarmten Vaginalflora ermöglichen und demzufolge Krankheiten im Vaginal- und Vulvenbereich weitgehend vorbeugen können.

In diesem Zusammenhang liegt der Erfindung die Aufgabe zugrunde, Tampons und wie Tampons anwendbare Mittel als Träger von vitalen Milchsäurebaktieren (Lacto-Bakterien) und/oder von Stoffen verfügbar zu haben, mit denen eine einfache Pflege und Stützung einer gesunden Vaginalflora oder deren Gesundung im Rahmen einer üblichen Pflege des femininen Intimbereichs ermöglicht ist. Solche Tampons oder dergleichen werden nachstehend als Lacto-Tampons und die vorstehenden Träger werden als Lacto-Träger bezeichnet. Tampons mit Gelatine schichten bzw. Lacto-Bakterien sind aus der WO-A-92/13577 und der EP-A-0,415 087 bekannt.

Aufgabe der Erfindung ist es somit, einen leicht herstellbaren und leicht anwendbaren Lacto-Tampon oder dergleichen vorzuschlagen, der zur Pflege und Stützung einer gesunden Vaginalflora einen möglichst intensiven Kontakt mit den Schleimhäuten im Vaginalbereich hält und dabei vitale Lacto-Bakterien und/oder sonstige Stoffe zur Gesundung oder Gesunderhaltung der Vaginalflora abgibt.

Die Aufgabe wird erfindungsgemäß mit den Merkmalen des Anspruches 1 gelöst.

Vorteilhafte Ausführungen nach der Erfindung ergeben sich aus den Merkmalen der Unteransprüche und der nachfolgenden Beschreibung. In der zugehörigen Zeichnung sind lediglich erfindungsgemäße Ausführungsbeispiele ohne jede Beschränkung nur schematisch dargestellt. Hierin zeigt:
- Figur 1: einen herkömmlichen Vaginaltampon, in perspektivischer Ansicht
- Figur 2: einen Lacto-Träger für einen erfindungsgemäßen Tampon ebenfalls in perspektivischer Ansicht, teilweise aufgebrochen
- Figur 3: eine Phase bei der Herstellung des Lacto-Trägers nach Fig. 2;
- Figur 4: der innere Teil eines Lacto-Trägers für einen erfindungsgemäßen Tampon in der Draufsicht
- Figur 5: einen weiteren Lacto-Träger für einen erfindungsgemäßen Tampon in perspektivischer Ansicht, teilweise aufgebrochen,
- Figur 6: eine Phase bei der Herstellung des Lacto-Trägers nach Fig. 5,
- Figur 7: ein zu einer Rolle aufgewickeltes Lacto-Trägerband mit einer Vielzahl von aneinanderhängenden Lacto-Trägern jeweils für einen erfindungsgemäßen Tampon,
- Figur 8: einen weiteren erfindungsgemäßen Tampon in perspektivischer Ansicht, teilweise aufgebrochen,
- Figur 9: den in eine Ebene ausgebreiteten Tampon nach Fig. 8 in perspektivischer Draufsicht, teilweise aufgebrochen,
- Figur 10: einen noch weiteren erfindungsgemäßen Tampon in perspektivischer Ansicht, teilweise angebrochen
- Figur 11: den in eine Ebene ausgebreiteten Tampon nach Fig. 10 in perspektivischer Draufsicht, teilweise aufgebrochen,
- Figur 12: einen noch weiteren erfindungsgemäßen Tampon in perspektivischer Ansicht, teilweise aufgebrochen,
- Figur 13: den in eine Ebene ausgebreiteten Tampon nach Fig. 12 in perspektivischer Draufsicht,
- Figur 14: einen noch weiteren erfindungsgemäßen Tampon in perspektivischer Ansicht, teilweise aufgebrochen,
- Figur 15: den in eine Ebene ausgebreiteten Tampon nach Fig. 14 in der Draufsicht, teilweise aufgebrochen
- Figur 16: eine Variante des Tampons nach Fig. 14 und 15 ebenfalls in der Draufsicht

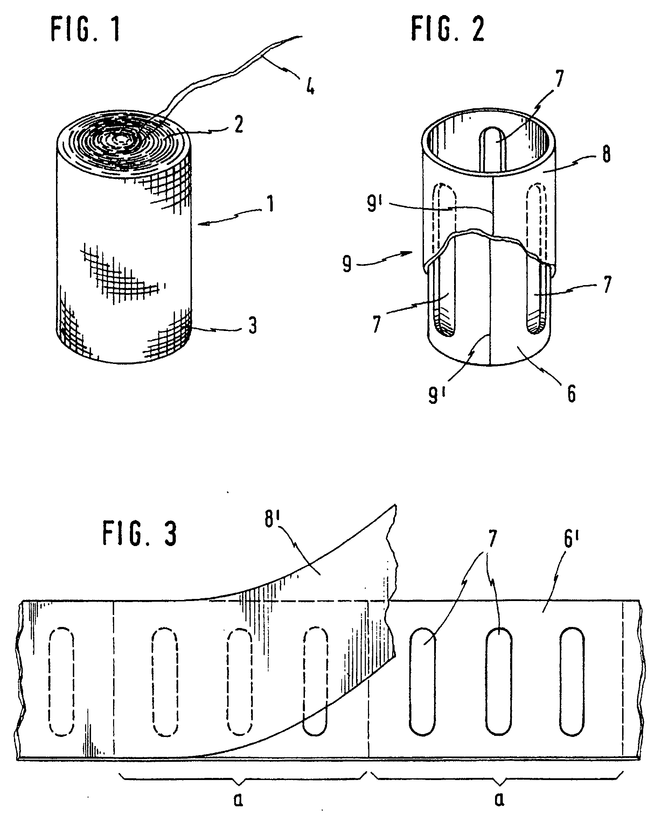

Fig. 1 zeigt in perspektivischer Darstellung einen herkömmlichen Vaginaltampon 1 zum Aufsaugen der Regelblutung. Der Tampon besteht aus einer hoch verdichteten, schneckenförmig ausgerollten und in Längsrichtung eng plissierten Wattebahn 2 innerhalb eines mit 3 bezeichneten, eng plissierten Vliesstoffes, Gittertülls oder dergleichen, der zum Halt der aufgerollten Wattebahn 2 als schlauchförmiger Mantel des Tampons 1 ausgebildet ist. An die Wattebahn ist eine Schnur 4 zum Herausziehen des Tampons nach seiner Benutzung angeschlossen.

Fig. 2 zeigt einen Lacto-Träger als ring- oder zylinderförmigen Körper, der z.B. einen herkömmlichen Tampon nach Fig. 1 mit oder ohne seinen plissierten Vliesstoffmantel umschließen kann.

Der Lacto-Träger nach Fig. 2 besteht erfindungsgemäß aus zwei dünnen, dicht übereinanderliegenden und fest aneinanderhaftenden Schichten 6 und 8 aus wasserlöslicher Gelatine oder dergleichen Polypeptide, wie sie z.B. für Medikamentenkapseln verwendet werden. Diese und vergleichbare Schichten werden nachstehend ohne jede Beschränkung als Gelatineschichten bezeichnet.

Im Beispielsfalle enthält die innere Gelatineschicht mehrere kammerartige Vertiefungen 7, die mit Abstand voneinander über den Umfang des Lacto-Trägers gleichmäßig verteilt angeordnet sind und sich in Längsrichtung des Lacto-Trägers erstrecken. Im Beispielsfalle sind drei gleichartige längliche Vertiefungen 7 vorgesehen. Die Anzahl der Vertiefungen und ihre Gestalt stellt für die Erfindung keine Beschränkung dar. Die längliche Anordnung in Längsrichtung des Lacto-Trägers ist gewählt, um diesen leicht aus einem flachen, bandförmigen Abschnitt in eine zylindrische oder ringförmige Form bringen zu können und die Einführung des Tampons zu erleichtern. Es ist wenigstens eine Vertiefung 7 vorhanden.

Die innere Gelatineschicht 6 ist von der flachen äußeren Gelatineschicht 8 überdeckt. Dabei sind die beiden Schichten derart fest und luft- sowie wasserdicht miteinander verbunden, daß die einzelnen kammerartigen Vertiefungen 7 jeweils dicht abgeschlossen sind.

In wenigstens eine Vertiefung 7 ist ein körniges Lyophilisat von Milchsäurebakterien (Lacto-Lyophilisat) eingebracht. Je nach Wahl können auch sämtliche Vertiefungen mit dem Lacto-Lyophilisat gefüllt sein. Es kann vorteilhaft sein, wenn wenigstens eine der Kammern 7 mit einem Hilfsstoff gefüllt ist, der z.B. das Wachstum der freigesetzten Lacto-Bakterien und ihren Kontakt mit den Vaginalschleimhäuten begünstigt und/oder die vorhandene natürliche Vaginalflora positiv beeinflußt. Hierbei kann es sich z.B. um Nährmedien für die Lacto-Bakterien, Milchsäure für die Unterstützung der natürlich vorhandenen Vaginalflora und um Inulin und Oligofruktosen handeln. Der Hilfsstoff, auch als Gemisch, kann auch in lyophiler oder lipophiler, gelatineunlöslicher, flüssiger Form vorhanden sein.

Die beiden einen Ring oder Zylinder bildenden Gelatineschichten 6 und 8 als Träger von Lacto-Lyophilisat und/oder vorgenannten Hilfsstoffen können einen Mantel 9 eines Wattetampons bilden oder können auch ohne diesen bei ausreichender Steifigkeit als ein Hohlkörper tamponartig verwendbar sein.

Vorteilhafterweise besitzt dabei jeweils die außenliegende Gelatineschicht 8 eine weitgehend hohe bzw. leichte Löslichkeit im feuchten Scheidenmilieu, um das Lacto-Lyophilisat nach der Einführung des Tampons möglichst kurzzeitig freigesetzt zu bekommen, während die innere Gelatineschicht 6 eine vergleichsweise geringe Löslichkeit aufweist oder während der Benutzungszeit auch im wesentlichen unlöslich sein kann. Hierbei kann es vorteilhaft sein, die Schichtdicke der äußeren Gelatineschicht 8 dünner als die der inneren Gelatineschicht 6 zu wählen, so daß die geringere Löslichkeit der inneren Gelatineschicht 6 z.B. auch allein über die Schichtdicke gesteuert ist.

Der Grund für die unterschiedlichen Löslichkeiten liegt darin, daß die Saugwirkung der Tamponwatte herkömmlicher Tampons (wenn der Lacto-Träger als Tamponmantel für Tamponwatte verwendet ist) während der Einwirkzeit der zugeführten Lacto-Bakterien weitgehend ausgeschaltet sein muß, um die Kontaktnahme der Lacto-Bakterien mit den Schleimhäuten nicht zu hindern oder zu erschweren.

Kommt keine Tamponwatte zum Einsatz, dient die innere Gelatineschicht als tragendes Gerüst des Lacto-Trägers, der tamponähnlich verwendbar ist. Hierbei kann die innere Gelatineschicht auch unlöslich sein. In Verbindung mit einem Bändchen wie bei einem herkömmlichen Tampon, kann dann die nicht oder nicht vollständig aufgelöste innere Gelatineschicht nach der Benutzung aus der Scheide herausgezogen werden.

Wird der Lacto-Träger als Mantel eines beispielsweise herkömmlichen Wattetampons verwendet, kann es vorteilhaft sein, die innere Gelatineschicht 6 des Lacto-Trägers an mindestens einer schmalen Stelle mit einer relativ hohen Wasserlöslichkeit zu versehen, so daß bei der Benutzung diese Stelle nach der Auflösung wie eine Bruchstelle wirkt und dadurch eine radiale Ausdehnung der Tamponwatte nach ihrer Feuchtigkeitsaufnahme möglich wird. Hierbei bleibt dann die Saugwirkung der Tamponwatte bis auf den relativ kleinen Bereich der Bruchstelle in der inneren Gelatineschicht 6 radial nach außen weitgehend abgeschirmt.

Es kann erfindungsgemäß auch von Vorteil sein, die Saugwirkung der Tamponwatte nach einer gewählten Behandlungszeit wieder weitgehend freizugeben. Hierzu ist die volle Auflösbarkeit der inneren Gelatineschicht entsprechend zeitlich eingestellt. Der Lacto-Tampon wirkt dann während einer anfänglichen Zeit als Mittel zur Gesundung oder Gesunderhaltung der Vaginalflora und anschließend in herkömmlicher Weise zur Aufnahme der Regelblutung. Der erfindungsgemäße Lacto-Tampon kann daher auch kurzzeitig vor Einsetzen der Regelblutung verwendet werden.

Fig. 3 zeigt lediglich prinzipiell, wie ein zweischichtiger Träger für Lacto-Lyophilisat und/oder die vorgenannten Hilfstoffe hergestellt werden kann.

In einen endlosen bandförmigen Gelatinestreifen 6' sind aufeinanderfolgend die Vertiefungen 7 eingeformt, in die mit einem nicht dargestellten Abfüllmechanismus Lacto-Lyophilisat und/oder die Hilfsstoffe getrennt eingefüllt werden. Anschließend wird ein zweiter endloser bandförmiger Gelatinestreifen 8' an den Gelatinestreifen 6' dicht angeschlossen, wobei die einzelnen Vertiefungen 7 mit den eingefüllten Stoffen jeweils dicht abgeschlossen werden.

Um die Lacto-Träger in die ring- oder zylinderförmige Gestalt entsprechend Fig. 2 zu bringen, werden Abschnitte "a" von dem doppelwandigen Gelatinestreifen 6', 8',abgetrennt und in die ring- oder zylinderförmige Gestalt entsprechend Fig. 2 gebracht, wobei die Enden eines Abschnittes zur Bildung einer Stoß- oder Nahtstelle 9 miteinander verklebt oder verschweißt werden.

Die die Vertiefungen 7 aufweisende Gelatineschicht 6 muß nicht notwendigerweise die innere und die abdeckende Gelatineschicht 8 muß nicht notwendigerweise die äußere Schicht sein, wie es der Lacto-Träger nach Fig. 2 zeigt. Die die Vertiefungen enthaltene Gelatineschicht kann auch die außen und die abdeckende Gelatineschicht 8 kann innen liegen, wobei dann auch die vorstehenden Löslichkeiten entsprechend vertauscht sind.

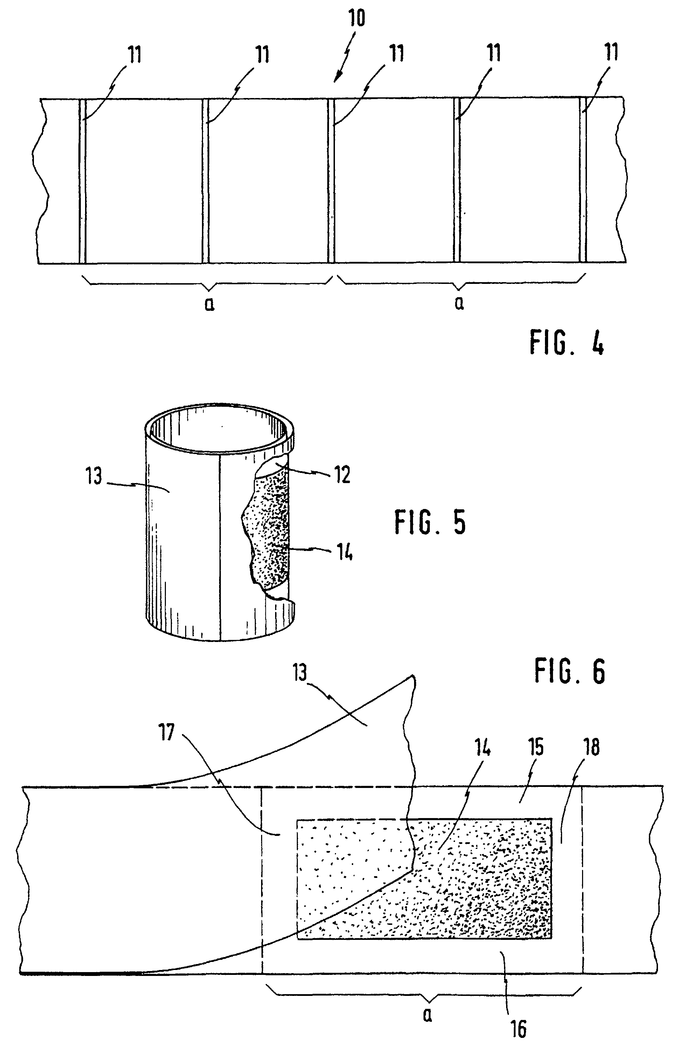

Fig. 4 zeigt einen inneren Gelatinestreifen 10, von dem, entsprechend Fig. 3, Abschnitte "a" jeweils zur Bildung eines Lacto-Trägers abgetrennt werden. Der Gelatinestreifen 10 besitzt mit Abstand aufeinanderfolgend quer verlaufende Streifen 11 aus einem Gelatinematerial mit einer relativ höheren Löslichkeit als das übrige Gelatinematerial.

Wird ein doppelwandiger Lacto-Träger unter Verwendung eines Abschnittes "a" des Gelatinestreifens 10 hergestellt, so bricht er bei der Benutzung nach einer Auflösung der schmalen Gelatinestreifen 11 auf und erlaubt dadurch die radiale Ausdehnung der Tamponwatte im Zentrum des Lacto-Trägers, wobei die Saugwirkung durch den nicht aufgelösten Abschnitt "a" des Gelatinestreifens 10 im wesentlichen blockiert bleibt.

Im Beispielfall unter Verwendung einer inneren Gelatineschicht entsprechend Fig. 2, hergestellt aus dem Gelatinestreifen nach Fig. 4, bricht die innere Gelatineschicht nach Auflösung zweier Gelatinestreifen 11 in zwei Halbschalen auf, die die Saugwirkung der Tamponwatte nach außen zumindest während einer vorbestimmten Zeitspanne blockieren.

Fig. 5 zeigt eine Variante eines erfindungsgemäßen hohlen Lacto-Trägers aus einer inneren Gelatineschicht 12 und einer äußeren Gelatineschicht 13. Im vorliegenden Fall besitzt weder die innere noch die äußere Gelatineschicht Vertiefungen. Stattdessen ist z.B. Lyophilisat in einem schmalen Streifen 14 aus einem körnigen Material mittig auf die eine der beiden flachen Gelatineschichten 12 oder 13 aufgetragen, wie Fig. 6 zeigt. Die jeweils andere Gelatineschicht dient als flache Abdeckschicht. Zum dichten Verschluß des Lyophilisatstreifens 14 zwischen den beiden Gelatineschichten 12 und 13 sind die Schichten an ihren längs verlaufenden unteren und oberen Rändern 15 und 16 sowie an quer verlaufenden Trennstellen 17 und 18 im Abstand "a" miteinander dicht verklebt oder verschweißt. Aus einem doppelwandigen Gelatineabschnitt der Länge "a" wird dann der Lacto-Träger gemäß Fig. 5 geformt, wobei die außenliegende Gelatineschicht wiederum eine vergleichsweise hohe Wasserlöslichkeit aufweist und die innenliegende Gelatineschicht eine vergleichsweise niedrigere Wasserlöslichkeit besitzt oder auch unlöslich ist, je nachdem, ob der Lacto-Träger ohne oder mit einem zentralen Wattetampon benutzt wird. Im letzteren Fall kann die innere Gelatineschicht auch entsprechend Fig. 4 ausgebildet sein.

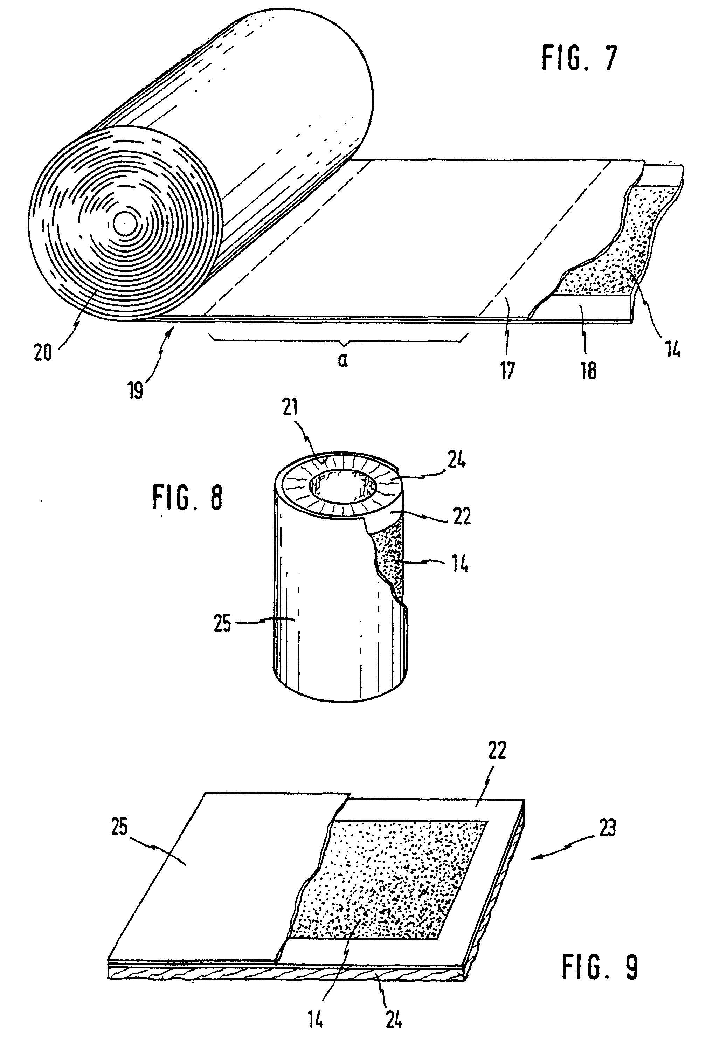

Es kann weiterhin vorteilhaft sein, einen endlosen doppelwandigen Gelatinestreifen aus einer Vielzahl von aufeinanderfolgenden Abschnitten "a" jeweils für einen Lacto-Träger beispielsweise entsprechend Fig. 2 oder 5 oder für weitere nachstehend beschriebene Lacto-Träger bzw. Lacto-Tampons in einer endlosen Bahn 19 herzustellen und diese zu einer Vorratsrolle 20 aufzurollen, wie es in Fig. 7 schematisch angedeutet ist. Zur Herstellung eines Lacto-Trägers wird von der Vorratsrolle ein Abschnitt "a" in der Länge des Umfangs eines Lacto-Trägers abgetrennt und hieraus der Lacto-Träger bzw. der Lacto-Tampon in eine zylindrische oder ringförmige Form gebracht.

Es kann auch vorteilhaft sein, an der Innenseite 21 der inneren Gelatineschicht 22 des Lacto-Trägers 23, nach Fig. 8 eine dünne Watteschicht 24 anzubringen. Die äußere Gelatineschicht 25 dient zur Abdeckung des Lacto-Lyophilisats 14, das z.B. entsprechend Fig. 6 auf die innere Gelatineschicht 22 streifenförmig aufgetragen ist.

Fig. 9 zeigt den Lacto-Träger 23 mit der inneren Watteschicht 24 entsprechend Fig. 8 in ausgebreiteter Form perspektivisch von oben, teilweise aufgebrochen..

Die dünne innere Watteschicht 24 besitzt praktisch keine wesentliche Saugwirkung und dient hauptsächlich zur Erhöhung der Stabilität des Lacto-Trägers. Es ist klar, daß die obige Lösung auch für die vorstehend beschriebenen Ausführungsbeispiele der Erfindung geeignet sind. Hierdurch wird der Lacto-Träger zu einem Lacto-Tampon mit geringem Watteanteil, der eine unbedeutende Saugwirkung aufweist.

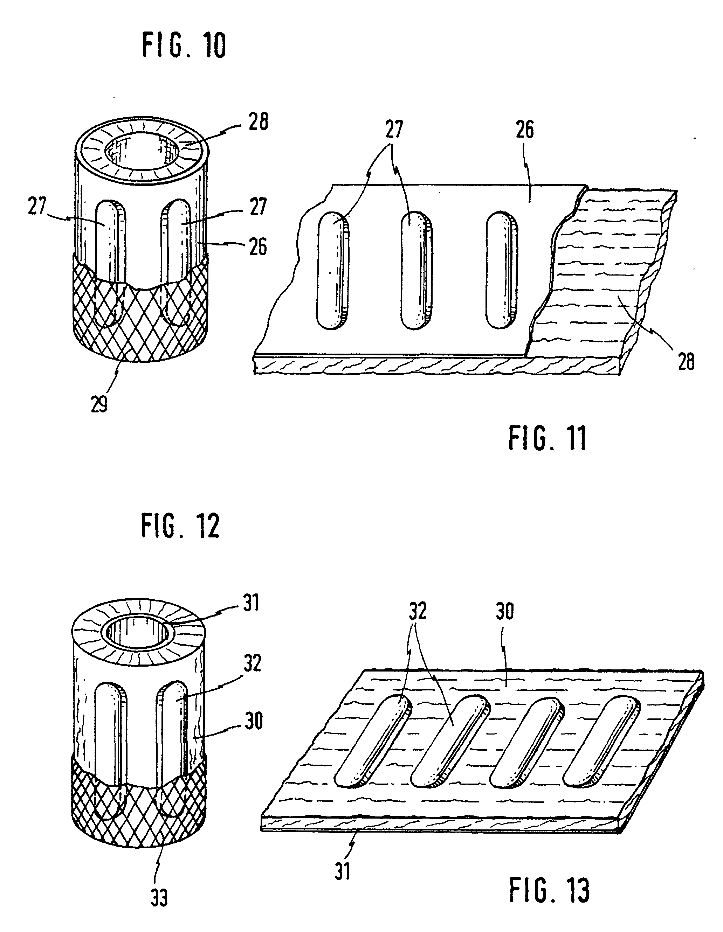

Fig. 10 zeigt noch einen weiteren erfindungsgemäßen Lacto-Tampon, der in Fig. 11 in eine Ebene aufgerollt ist.

Auf der Oberseite einer einzigen Gelatineschicht 26 sind in Abständen aufeinanderfolgend Gelatinekapseln 27 fest angebracht, die z.B. Lacto-Lyophilisat und/oer Hilfsstoffe der vorstehenden Art enthalten. An der Unterseite der Gelatineschicht 26 befindet sich eine ralativ dünne Watteschicht 28. Diese Watteschicht kann aber auch entfallen, wenn der verbleibende Lacto-Träer eine ausreichende Stabilität besitzt um tamponartig verwendbar zu sein.

Die einzelnen Gelatinekapseln 27 ersetzten hier die äußere Gelatineschicht im Sinne der vorstehenden Ausführungsbeispiele.

Es kann von Vorteil sein, den erfindungsgemäßen Lacto-Tampon nach Fig. 10 in einem grobmaschigen schlauchförmigen Gittertüllmantel oder dergleichen, vergleichbar wie bei herkömmlichen Tampons, einzubringen. Die Grobmaschigkeit ist von Vorteil, um den Kontakt der Lacto-Bakterien mit den Schleimhäuten nicht zu behindern.

Fig. 12 zeigt noch einen weiteren erfindungsgemäßen Lacto-Tampon, der in Fig. 13 in eine Ebene ausgerollt ist.

Der Lacto-Tampon besteht hier aus einer schmalen Watteschicht 30 praktisch ohne Saugwirkung. Die Watteschicht ist von einer schwer löslichen oder unlöslichen Gelatineschicht 31 getragen. Auf der Oberseite der Watteschicht 30 sind in Abständen leicht lösliche Gelatinekapseln 32 gehalten, die z.B. Lacto-Lyophilisat und/oder Zusatzstoffe enthalten.

Zur Stabilisierung des Lacto-Tampons mit der außenliegenden Watteschicht 30 nebst den von ihr außen getragenen Gelatinekapseln 32 und der inneren Gelatineschicht 31 befindet sich dieser innerhalb eines möglichst weitmaschigen gittertüllartigen Mantels 33. Die dünne Watteschicht 30 dient hier bei der Benutzung als Brutstätte für die durch die gelösten Kapseln freigesetzten Lacto-Bakterien, von der aus sie in Kontakt mit den Schleimhäuten tritt.

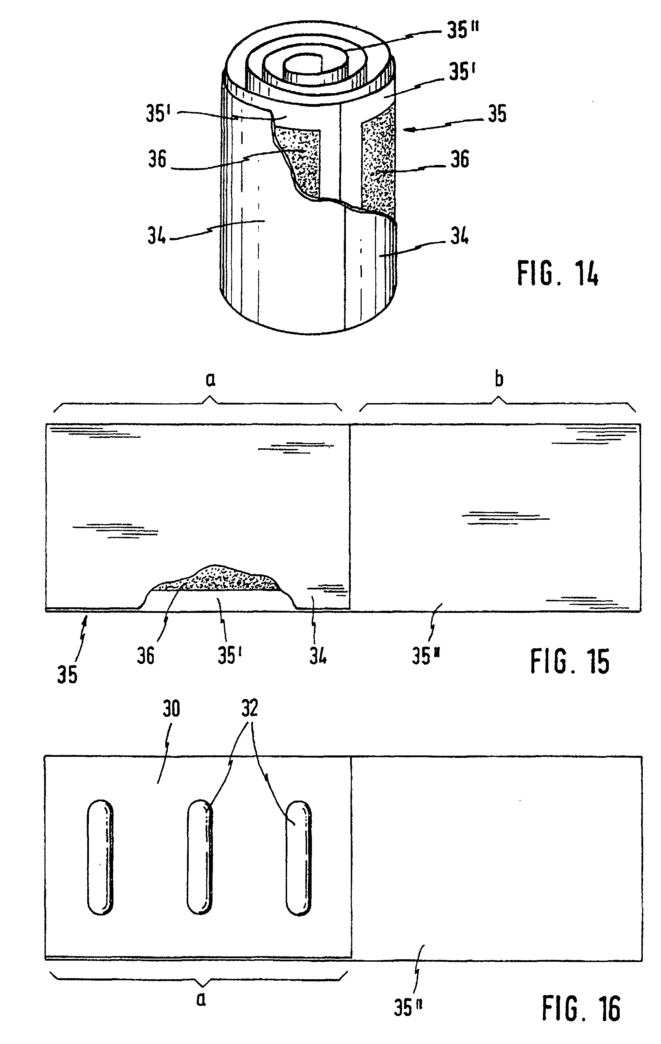

Schließlich zeigt Fig. 14 noch einen erfindungsgemäßen Lacto-Tampon, der in Fig. 15 in eine Ebene ausgerollt ist.

Zwischen einer äußeren leicht löslichen Gelatineschicht 34 und einer inneren schwer löslichen oder unlöslichen Gelatineschicht 35 ist beispielsweise gemäß Fig. 6 Lyophilisat 36 dicht eingeschlossen. Die äußere Gelatineschicht 34 erstreckt sich über den gesamten äußeren Umfang "a" des Lacto-Tampons und bildet mit dem von ihr abgedeckten Abschnitt 35' der schwerer löslichen inneren Gelatineschicht 35 einen geschlossenen zylindrischen oder ringförmigen Körper. Die innere gegebenenfalls unlösliche Gelatineschicht 35 überragt die äußere Gelatineschicht 34 mit einem anschließenden Abschnitt 35'' um ein gewähles Maß "b". Der Gelatineabschnitt "b" wird zur Bildung des Tampons spiralartig unter gewählter radialer Vorspannung aufgerollt, wie es schematisch in Fig. 14 in der Perspektive gezeigt ist. Erst nach Auflösung der äußeren Gelatineschicht 34 bei der Benutzung kommt die radiale Vorspannung des inneren Gelatineabschnittes 35'' gegenüber dem Abschnitt 35' zur Wirkung.

Die Dicke und die elastische Spannung der inneren Gelatineschicht 35 kann so gewählt sein, daß damit die radial nach außen gerichtete Vorspannung im gewünschten Maße gewählt werden kann, so daß bei Benutzung nach Auflösung der äußeren leicht löslichen Gelatineschicht 34 ein intensiver Kontakt der freigesetzten Lacto-Bakterien an den Schleimhäuten der Vagina sichergestellt ist.

Es ist klar, daß die vorstehend angegebenen Ausführungsbeispiele entsprechend mit einer inneren erfindungsgemäßen Gelatinespirale der vorstehenden Art ausgerüstet werden können.

Fig. 16 zeigt noch eine Variante, nach der statt der äußeren Gelatineschicht 34 eine äußere Watteschicht 30 entsprechend Fig. 12, 13 vorhanden ist, die Träger, z.B. von Gelatinekapseln 32 für Lacto-Lyophilisat und/oder Zusatzstoffe ist, wobei die Watteschicht mit den Gelatinekapseln in einem weitmaschigen Gittertüll 33 entsprechend Fig. 12 gehalten ist.

Die Lacto-Bakterien können aus der Gruppe bestehend aus Lactobacillus acidophilus, Lactobacillus Casei, Bifidobacterium bifidum, Streptococcus lactis und Lactobacillus Reuteri ausgewählt sein. Vorteilhafterweise wird ein Gemisch von mehreren dieser Bakterien verwendet.

Die Erfindung ist auf die Ausführungsbeispiele nicht beschränkt, die den Fachmann im Rahmen der Erfindung zu weiteren Abwandlungen ohne weiteres anregen kann.

## Patentansprüche

1. Vaginaltampon als Träger von vitalen Milchsäurebakterien (Lacto-Bakterien) und/oder Hilfsstoffen zur Pflege oder Regenerierung einer Vaginalflora ,
**dadurch gekennzeichnet,**
**daß** die Lacto-Bakterien und/oder Hilfsstoffe zwischen zwei dünnen Schichten aus Gelatine luft- und wasserdicht eingeschlossen sind, wobei die äußere Gelatineschicht eine ausgewählte hohe und die innere Schicht eine ausgewählte niedrige Wasserlöslichkeit besitzen oder wenigstens zeitlich begrenzt unlöslich ist.

2. Vaginaltampon nach Anspruch 1, **dadurch gekennzeichnet, daß** die beiden Gelatineschichten gemeinsam einen äußeren zylindrischen Mantel für einen Wattetampon bilden.

3. Vaginaltampon nach Anspruch 2, **dadurch gekennzeichnet, daß** die äußere Gelatineschicht kapselförmig ausgebildet ist.

4. Vaginaltampon nach Anspruch 2 oder 3, **dadurch gekennzeichnet, daß** die Innenseite der inneren Gelatineschicht Träger einer inneren Watteschicht ist.

5. Vaginaltampon nach einem der vorstehenden Ansprüche 2 bis 4, **dadurch gekennzeichnet, daß** die innere Gelatineschicht Träger einer Watteschicht im wesentlichen ohne Saugkraft ist.

6. Vaginaltampon nach einem der vorstehenden Ansprüche, **dadurch gekennzeichnet, daß** ein freier Endabschnitt der inneren Gelatineschicht zu einer zentral liegenden Spirale gedreht ist, die unter radialer elastischer Vorspannung steht, die nach wenigstens teilweiser Auflösung der äußeren Gelatineschicht freigesetzt ist.

7. Vaginaltampon nach einem der vorstehenden Ansprüche, **dadurch gekennzeichnet, daß** die innere Gelatineschicht an wenigstens einer vorbestimmten Stelle eine relativ hohe Löslichkeit aufweist.

## Claims

1. A vaginal tampon as a carrier of living lactic acid bacteria (lacto bacteria) and/or auxiliary substances for treating or regenerating the vaginal flora, **characterized in that**
the lacto bacteria and/or auxiliary substances are enclosed between two thin gelatine layers in an air and water-tight manner whereby the outer gelatine layer has a selected high and the inner gelatine layer has a selected low water solubility or is at least restrictedly insoluble for a certain period of time.

2. Vaginal tampon according to claim 1, **characterized in that** the two gelatine layers together form an outer cylindrical casing for a cotton-wool tampon.

3. Vaginal tampon according to claim 2, **characterized in that** the outer gelatine layer is adapted to be in a form of a capsule.

4. Vaginal tampon according to claim 2 or 3, **characterized in that** the inside of the inner gelatine layer is the carrier of an inner cotton-wool layer.

5. Vaginal tampon according to one of the above mentioned claims 2 to 4, **characterized in that** said inner gelatine layer is the carrier of a cotton-wool layer which is essentially without suction force.

6. Vaginal tampon after one of the above mentioned claims, **characterized in that** a free ending section of said inner gelatine layer is rolled up as a central spiral, which is subjected to radial elastic initial tension, which is released after an at least partial dissolution of said outer gelatine layer.

7. Vaginal tampon according to one of the above mentioned claims, **characterized in that** said inner gelatine layer comprises at least at one predetermined location a relatively large solubility.

## Revendications

1. Tampon vaginal comme porteur de bactéries d'acide lactique vitales (lactobactéries) et/ou de substances auxiliaires pour le soin ou la régénération de la flore vaginale, **caractérisé en ce que** les lactobactéries et/ou les substances auxiliaires sont incluses entre deux minces couches de gélatine de façon étanche à l'air et à l'eau, sachant que la couche de gélatine extérieure possède une solubilité élevée déterminée et la couche intérieure possède une faible solubilité déterminée ou est au moins insoluble pendant un temps limité.

2. Tampon vaginal selon la revendication 1, **caractérisé en ce que** les deux couches de gélatine forment ensemble un manteau cylindrique extérieur pour un tampon d'ouate.

3. Tampon vaginal selon la revendication 2, **caractérisé en ce que** la couche de gélatine extérieure est réalisée en forme de capsule.

4. Tampon vaginal selon l'une des revendications 2 ou 3, **caractérisé en ce que** le côté intérieur de la couche de gélatine intérieure est le support d'une couche d'ouate intérieure.

5. Tampon vaginal selon l'une des revendications 2 à 4, **caractérisé en ce que** la couche de gélatine intérieure est le support d'une couche d'ouate essentiellement sans pouvoir d'aspiration.

6. Tampon vaginal selon l'une des revendications précédentes, **caractérisé en ce qu'**une section d'extrémité libre de la couche de gélatine intérieure forme par rotation une spirale située en position centrale, qui se trouve sous une prétention élastique radiale, qui est relâchée après la dissolution au moins partielle de la couche de gélatine extérieure.

7. Tampon vaginal selon l'une des revendications précédentes, **caractérisé en ce que** la couche de gélatine intérieure présente une solubilité relativement élevée à au moins un endroit prédéterminé.
